# EUROPÄISCHE PATENTSCHRIFT

(11) **EP 0 748 373 B1**
(45) Veröffentlichungstag und Bekanntmachung des Hinweises auf die Patenterteilung: **27.12.2000**
(21) Anmeldenummer: 95912181.5
(22) Anmeldetag: 01.03.1995
(51) Int. Cl.: C12N 1/36, C12N 1/26, C02F 3/34

(54) **VERFAHREN FÜR DIE ZÜCHTUNG VON MIKROORGANISMEN**
MICRO-ORGANISM CULTURE METHOD
PROCEDE DE CULTURE DE MICRO-ORGANISMES

(30) Priorität: 02.03.1994 DE 4406843; 01.03.1995 DE 19507103
(43) Veröffentlichungstag der Anmeldung: 18.12.1996
(73) Patentinhaber: Bitop Gesellschaft für biotechnische Optimierung MbH, D-58453 Witten (DE)
(72) Erfinder: BARTHOLMES, Peter, D-58456 Witten (DE); KAUFMANN, Michael, D-44388 Dortmund (DE); SCHWARZ, Thomas, D-42799 Leichlingen (DE)
(74) Vertreter: Schneiders, Josef, Dipl.-Ing.
(86) Internationale Anmeldenummer: EP9500752
(87) Internationale Veröffentlichungsnummer: WO9523845

(56) Entgegenhaltungen:
- FR-A- 2 665 180
- GB-A- 2 010 327

## Beschreibung

Die Erfindung betrifft ein Verfahren für die Züchtung von Mikroorganismen, die zum Abbau von bestimmten Schadstoffen geeignet sind, bei welchem vorhandene Mikroorganismen in einer wachstumsfördernden Nährlösung kultiviert, chemisch und/oder physikalisch mutagen wirkenden Einflüssen ausgesetzt, unter wachstumsfördernden Bedingungen stabilisiert und unter dem Einfluß der Schadstoffe unter wachstumsbeschränkenden Bedingungen selektiert werden, wobei diese selektierten Mikroorganismen an den Anfang des Prozesses zurückgeführt werden.

Die Nutzung von Mikroorganismen zum biologischen Abbau von Schadstoffen findet einen wichtigen Einsatz in der Altlastensanierung sowie in der Abwässer- und Luftreinigung, bei denen die Schadstoffe durch die Mikroorganismen zersetzt oder in andere Stoffe umgewandelt werden, deren Entsorgung unproblematischer ist. Für den effizienten biologischen Abbau von Schadstoffen geeignete Mikroorganismen müssen über die spezifische Abbaufähigkeit hinaus eine optimale Schadstofftoleranz und eine möglichst hohe Teilungsrate sowie genetische Stabilität aufweisen.

Zur Züchtung von Bakterienmutanten, die zum Abbau von Schadstoffen befähigt sind, wird in DE 41 24 900 A1 ein Verfahren offenbart, bei dein die Bakterien unter nichtwachstumsbeschränkenden Bedingungen gezüchtet, einer mutationserzeugenden UV-Strahlung ausgesetzt und die mutierten Bakterien dann unter wachstumsbeschränkenden Bedingungen mit dem Schadstoff zusammengebracht werden. Gegebenenfalls könnten die Mikroorganismen aus der Selektionsstufe in einen ersten Reaktor überführt werden, in dem sie stabilisiert werden, um anschließend in der Mutationsstufe einer erneuten UV-Strahlung ausgesetzt zu werden.

Es hat sich aber gezeigt, daß das Verfahren nach dein Stand der Technik nicht zur Entwicklung solcher Mikroorganismenpopulationen geeignet ist, die organische Verbindungen und insbesondere komplexe Verbindungen abbauen können, weil eine bzw. zwei mutagene Behandlungen nicht hinreichen, um die Etablierung einer zum Abbau komplexer organischer Verbindungen geeigneten Mikroorganismenpopulation in vertretbarer Zeit zu erzielen. Dies ist zunächst ein prinzipielles statistisches Problem, welches mit großer Wahrscheinlichkeit damit zusammenhängt, daß zum Abbau komplexer organischer Schadstoffe mehrere Abbaureaktionen erforderlich sind, die sequentiell verlaufen und bei denen Zwischenprodukte erzeugt werden, die unter Umständen für die Mikroorganismen toxischer sind als das Ausgangsprodukt. Die Zusammensetzung des Selektionsmediums ändert sich also im Laufe der Zeit, so daß sich die Mikroorganismenpopulation bezüglich der Abbaufähigkeit und der Schadstofftoleranz immer wieder anpassen muß. Diese Anpassung nimmt einige Zeit in Anspruch und führt dazu, daß der Prozeß insgesamt länger dauert und daher unwirtschaftlich wird.

Das aus dem Stand der Technik vorbekannte Verfahren erfordert außerdem einen erheblichen apparativen Aufwand, weil Kultivierung, mutagene Behandlung und Stabilisierung der Mikroorganismenpopulation in unterschiedlichen Reaktoren durchgeführt werden. Darüber hinaus werden die Mikroorganismen nach der Stabilisierung in einem nährstoffreichen Medium zum Animpfen des schadstoffhaltigen Selektionsmediums direkt in den Selektionsfermenter überführt. So kann es geschehen, daß in der der Selektion unterworfenen Biomasse noch erhebliche Anteile an Nährsubstanzen enthalten sind, die in den Selektionsfermenter eingeschleppt worden sind und die die Selektion nachteilig beeinflussen, weil diese Nährsubstanzen von den Mikroorganismen bevorzugt als Nährstoffquelle genutzt werden. Die Selektion der schadstoffabbauenden Mikroorganismen wird also erst einsetzen, wenn die eingeschleppten Nährsubstanzen verbraucht sind. Dies führt zunächst zu einer positiven Selektion der Mikroorganismen, die den Schadstoff nicht abbauen können, was eine zusätzliche Verzögerung des gesamten Selektionsprozesses zur Folge hat.

Es ist deshalb Aufgabe der Erfindung, das Verfahren der eingangs genannten Art dahingehend weiterzubilden, daß einerseits die Kultivierung und Mutation unter wachstumsfördernden Bedingungen und andererseits die Selektion unter wachstumsbeschränkenden Bedingungen exakt kontrollierbar bleiben. Hierdurch soll es ermöglicht werden, mit geringerem apparativem Aufwand den natürlichen Evolutionsprozeß möglichst eng und gezielt zu kanalisieren, um auf diese Weise möglichst schnell Mikroorganismen mit den gewünschten Eigenschaften züchten zu können.

Zur Lösung dieser Aufgabe schlägt die Erfindung ausgehend vom Verfahren der eingangs genannten Art vor,
- daß die Kultivierung, die mutagene Behandlung und die Stabilisierung simultan in einem Mutationsfermenter vorgenommen werden, der ein nährstoffreiches Grundmedium enthält,
- daß die Selektion in einem Selektionsfermenter erfolgt, der ein nährstoffarmes, mit dem Schadstoff angereichertes Grundmedium enthält,
- daß dem Mutationsfermenter kontinuierlich in kleinen Mengen Biomasse entnommen wird, die von dem nährstoffreichen Grundmedium des Mutationsfermenters befreit und in den Selektionsfermenter überführt wird,
- daß dem Selektionsfermenter kontinuierlich in kleinen Mengen Biomasse entnommen wird, die von dem nährstoffarmen Grundmedium des Selektionsfermenters befreit und in den Mutationsfermenter überführt wird,
- daß das gesamte Verfahren nach Art eines sich wiederholenden Kreislaufes kontinuierlich in alternierenden Selektions- und Mutationsphasen abläuft,
   wobei dem Mutationsfermenter einerseits und dem Selektionsfermenter andererseits rechnergesteuerte Regelkreise zugeordnet sind, durch welche die Fermentationsparameter in beiden Fermentern unabhängig voneinander einstellbar sind.

Das Verfahren gemäß der Erfindung arbeitet in besonders vorteilhafter Weise mit einem Doppelfermentersystem, in welchem die beiden Prinzipien der Evolution - Selektion und Mutation - einzeln optimiert stattfinden können. Dabei werden die Mikroorganismen in beiden Fermentern, welche sich im Betrieb gegenseitig beimpfen, kontinuierlich in alternierenden Selektions- und Mutationsphasen kultiviert. Im Mutationsfermenter kann durch ultraviolette Bestrahlung und/oder Beigabe von mutagenen Chemikalien die Mutationsrate in der Kultur drastisch erhöht werden, während im Selektionsfermenter den geeigneten Mutanten gezielt zum Schadstoffabbau geeignete Bedingungen verschafft werden. Durch wiederholte mutagene Behandlungen wird die genetische Vielfalt der Mikroorganismenpopulation erhöht, so daß sich diese bezüglich Abbaufähigkeit und Schadstofftoleranz schneller anpassen kann, wenn sich die Bedingungen im Selektionsreaktor, also das Verhältnis der Mengen an Zwischenprodukten, im Laufe des Abbauprozesses ändert. So erfährt die Entwicklung der Mikroorganismenpopulation durch den Einsatz von Mutation und Selektion in einem zyklischen Prozeß eine drastische Beschleunigung gegenüber der einzelnen Mutation bzw. Selektion nach dem Stand der Technik.

Das Verfahren gemäß der Erfindung macht es somit möglich, verhältnismäßig schnell ganze Biozönosen auf speziell definierte Abbauleistungen und/oder Toleranzen hin zu generieren oder zu spezialisieren. Die Vorteile des Verfahrens liegen in der quasinatürlichen Erzeugung der Mikroorganismen durch gezielt gesteuerte Evolution. Das minder:, im Gegensatz zu gentechnisch manipulierten Mikroorganismen, das Risiko der Entstehung unwirksam gewordener Revertanten. Der Einsatz rechnergesteuerter Regelkreise erlaubt es, definierte chemische und verfahrenstechnische Bedingungen reproduzierbar einzustellen und auf diese Weise für alle Einzelprozesse die jeweils optimalen Bedingungen zu finden.

Eine besonders vorteilhafte Ausführungsform des Verfahrens gemäß der Erfindung sieht vor, daß der Selektionsfermenter (Fermenter 2) toxinstatisch arbeitet, d.h. daß im Laufe des Prozesses die Schadstoffkonzentration konstant bleibt. Dies hat einerseits den Vorteil, daß der Selektionsdruck über den gesamten Prozeß hinweg konstant aufrechterhalten wird, so daß nur solche Mikroorganismen wachsen können, die Toleranz gegenüber dem Schadstoff aufweisen bzw. den Schadstoff abbauen können. Andererseits hat es ebenfalls den Vorteil, daß sich in der Mikroorganismenpopulation schneller ein biozönotisches Gleichgewicht einstellt.

In einer weiteren bevorzugten Ausführungsform der Erfindung arbeitet der Selektionsfermenter (Fermenter 2) turbidostatisch. In diesem Fall wird der Selektionsprozeß hauptsächlich über die Biomasse im Selektionsfermenter gesteuert.

Vorzugsweise werden bei der Überführung von Biomasse von einem Fermenter in den anderen die Grundmedien der Biomasse ausgetauscht. Dieser Austausch der Grundmedien wird zweckmäßig in zwischen den Fermentern angeordneten Dialysestrecken vorgenommen. Auf diese Weise wird verhindert, daß Medium aus einem Fermenter in den anderen gelangt, wodurch den Mikroorganismen Nährstoffe angeboten würden, die gegenüber den Schadstoffen bevorzugt benutzt werden, was zu einer Verzögerung des Selektionsprozesses führt. Darüber hinaus wird durch die schonende Anpassung an das neue Medium, die in der Dialysestrecke stattfindet, erreicht, daß die "lag"-Phase bei jedem Fermenterwechsel verkürzt und so der gesamte Prozeß beschleunigt wird. Ein weiterer Vorteil besteht darin, daß durch den Austausch des Mediums zwischen dem Selektions- und dem Mutationsfermenter UV-absorbierende Substanzen entfernt werden, die im Selektionsmedium enthalten oder im Rahmen des mikrobiellen Stoffwechsels hergestellt und in das Medium ausgeschieden worden sind und die bei Einsatz von UV-Strahlung als Mutagen die Mutationseffizienz mindern können.

In einer bevorzugten Ausführungsform des erfindungsgemäßen Verfahrens enthält das Grundmedium (Grundmedium 1) des Mutationsfermenters (Fermenter 1) den abzubauenden Schadstoff. Der Mutationsfermenter arbeitet zweckmäßig toxinstatisch.

In einer weiteren bevorzugten Ausführungsform der Erfindung arbeitet der Mutationsfermenter (Fermenter 1) turbidostatisch.

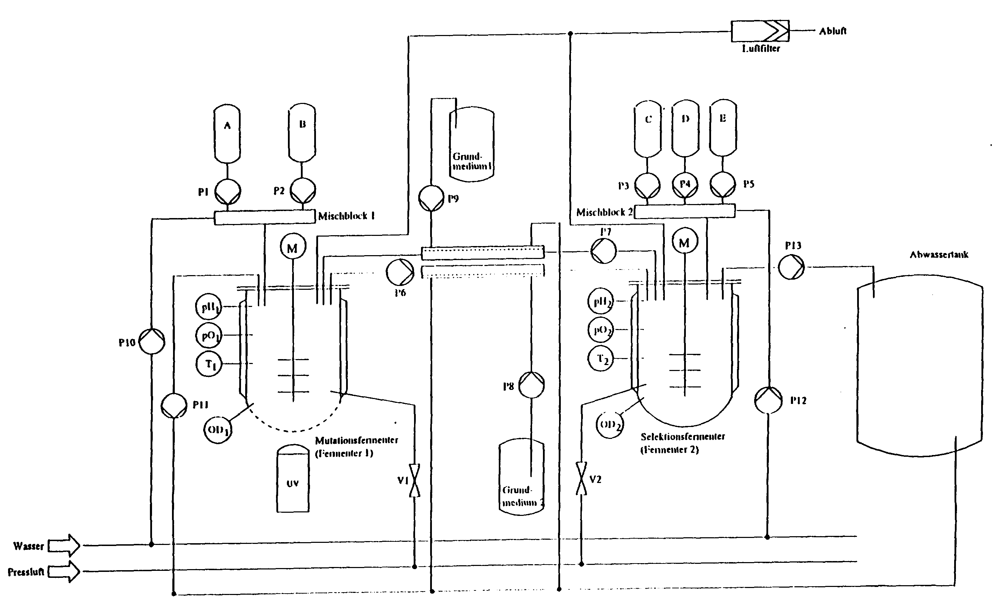

Ein Ausführungsbeispiel des Verfahrens gemäß der Erfindung wird im folgenden anhand der beigefügten Zeichnung erläutert, die schematisch den Aufbau einer zur Durchführung des Verfahrens geeigneten Anlage darstellt.

Die in der Zeichnung dargestellte Anlage weist zwei Fermentationsgefäße mit einer Kapazität von einem bis zwei Litern auf, die kontinuierlich betrieben werden können, nämlich den Mutationsfermenter (Fermenter 1) und den Selektionsfermenter (Fermenter 2). Beide Fermenter sind mit folgender Standardsensorik ausgestattet: pH₁ und pH₂ für die pH-Werte, pO₁ und pO₂ für die Sauerstoffgehalte, T₁ und T₂ für die Temperatur und OD₁ und OD₂ für die optische Dichte. Zusätzlich befindet sich in beiden Fermentern oder im Bypass beider Fermenter geeignete Sensorik zur Erfassung der aktuellen Toxin ("Selektionssubstrat")-Konzentrationen. Der Fermenter 2 kann über eine Pumpe P6 und eine nachgeschaltete Dialysestrecke, in der eine schonende Medienadaption erreicht wird, mit Mikroorganismen aus dem Fermenter 1 kontinuierlich angeimpft werden. Ebenso wird der Fermenter 1 über eine Pumpe P₇ und eine nachgeschaltete Dialysestrecke mit Mikroorganismen aus dem Fermenter 2 angeimpft. Pumpen P₈ und P₉ versorgen die Dialysestrecken mit Grundmedium. Beide Fermenter werden mit Preßluft über Ventile V₁ und V₂ begast und sind mit regelbaren Rührwerken M ausgestattet. In den Dialysestrecken werden die in den jeweils anderen Fermenter zu überführenden Mikroorganismen von dem Grundmedium des Herkunfts-Fermenters befreiz und mit dem Grundmedium des Ziel-Fermenters angereichert.

Der Mutationsfermenter (Fermenter 1) weist eine Bestrahlungsmöglichkeit mittels UV auf. Die Strahlenpassage erfolgt durch ein Quarzfenster. Der Fermenter 1 wird über einen Mischblock 1 sowie über Pumpen 10 (Wasser), P₁ (Medienkonzentrat) und P₂ (Nährsubstrat) mit Medium versorgt. Eine Pumpe P₁₁ sorgt durch pegelgerechtes Abpumpen in den Abwassertank für ein konstantes Volumen der Fermentationsbrühe.

Die Medienzufuhr in den Selektionsfermenter (Fermenter 2) erfolgt über einen Mischblock 2 sowie Pumpen P₁₂ (Wasser), P₃ (Medienkonzentrat), P₄ (Nährsubstrat) und P₅ ("Selektionssubstrat"). Eine Pumpe P₁₃ sorgt durch pegelgerechtes Abpumpen in einen Sammeltank für konstantes Volumen der Fermentationsbrühe.

Das System ist modular aufgebaut und erlaubt den Einsatz verschiedenster Typen von Selektionsreaktoren.

Sämtliche Pumpen sind für Dauerbetrieb ausgelegt und über geeignete Schnittstellen mit einem Prozeßleitsystem regelbar. Die Abwassertanks haben eine Kapazität von je 200 Litern. Der Lufteintrag, die Rührerdrehzahl, die Basen- bzw. Säurenzufuhr und die Temperierung werden von beiden Fermentersystemen autark und nicht über das Prozeßleitsystem geregelt.

## Patentansprüche

1. Verfahren für die Züchtung von Mikroorganismen, die zum Abbau von bestimmten Schadstoffen geeignet sind, bei welchem vorhandene Mikroorganismen in einer wachstumsfördernden Nährlösung kultiviert, chemisch und/oder physikalisch mutagen wirkenden Einflüssen ausgesetzt, unter wachstumsfördernden Bedingungen stabilisiert und unter dem Einfluß der Schadstoffe unter wachstumsbeschränkenden Bedingungen selektiert werden, wobei diese selektierten Mikroorganismen an den Anfang des Prozesses zurückgeführt werden,
**dadurch gekennzeichnet,**
daß die Kultivierung, die mutagene Behandlung und die Stabilisierung simultan in einem Mutationsfermenter Fermenter 1) vorgenommen werden, der ein nährstoffreiches Grundmedium (Grundmedium 1) enthält,
daß die Selektion in einem Selektionsfermenter (Fermenter 2) erfolgt, der ein nährstoffarmes, mit dem Schadstoff angereichertes Grundmedium (Grundmedium 2) enthält,
daß dem Mutationsfermenter (Fermenter 1) kontinuierlich in kleinen Mengen Biomasse entnommen wird, die von dem nährstoffreichen Grundmedium (Grundmedium 1) des Mutationsfermenters (Fermenter 1) befreit und in den Selektionsfermenter (Fermenter 2) überführt wird,
und daß dem Selektionsfermenter (Fermenter 2) kontinuierlich in kleinen Mengen Biomasse entnommen wird, die von dem nährstoffarmen Grundmedium (Grundmedium 2) des Selektionsfermenters (Fermenter 2) befreit und in den Mutationsfermenter (Fermenter 1) überführt wird,
daß das gesamte Verfahren nach Art eines sich wiederholenden Kreislaufes kontinuierlich in alternierenden Selektions- und Mutationsphasen abläuft,
wobei dem Mutationsfermenter (Fermenter 1) einerseits und dem Selektionsfermenter (Fermenter 2) andererseits rechnergesteuerte Regelkreise zugeordnet sind, durch welche die Fermentationsparameter in beiden Fermentern unabhängig voneinander einstellbar sind.

2. Verfahren nach Anspruch 1, dadurch gekennzeichnet, daß der Selektionsreaktor toxinstatisch arbeitet.

3. Verfahren nach Anspruch 1, dadurch gekennzeichnet, daß der Selektionsreaktor turbidostatisch arbeitet.

4. Verfahren nach Anspruch 1, 2 oder 3, dadurch gekennzeichnet, daß bei der Überführung von Biomasse von einem Fermenter in den anderen die Grundmedien der Biomasse ausgetauscht werden.

5. Verfahren nach Anspruch 4, dadurch gekennzeichnet, daß der Austausch der Grundmedien in zwischen den Fermentern angeordneten Dialysestrecken vorgenommen wird.

6. Verfahren nach einem oder mehreren der vorangegangenen Ansprüche, dadurch gekennzeichnet, daß das Grundmedium

7. Verfahren nach Anspruch 6, dadurch gekennzeichnet, daß der Mutationsfermenter (Fermenter 1) toxinstatisch arbeitet.

8. Verfahren nach Anspruch 6, dadurch gekennzeichnet, daß der Mutationsfermenter (Fermenter 1) turbidostatisch arbeitet.

## Claims

1. A method of cultivating micro-organisms which are suitable for breaking down certain harmful substances, in which micro-organisms present are cultivated in a growth-promoting nutrient solution, exposed to influences which have a chemically and/or physically mutagenic action, stabilised under growth-promoting conditions and selected under the influence of the harmful substances under growth-limiting conditions, wherein said selected micro-organisms are recycled to the beginning of the procedure, characterised in that
cultivation, mutagenic treatment and stabilisation are implemented simultaneously in a mutation fermenter (fermenter 1) which contains a nutrient-rich base medium (base medium 1),
selection is effected in a selection fermenter (fermenter 2) which contains a low-nutrient base medium enriched with the harmful substance (base medium 2),
biomass is continuously removed in small amounts from the mutation fermenter (fermenter 1), which biomass is freed of the nutrient-rich base medium (base medium 1) of the mutation fermenter (fermenter 1) and transferred into the selection fermenter (fermenter 2),
and biomass is continuously removed in small amounts from the selection fermenter (fermenter 2), which biomass is freed of the low-nutrient base medium (base medium 2) of the selection fermenter (fermenter 2) and transferred into the mutation fermenter (fermenter 1), and
the entire method takes place continuously in the manner of a repetitive circulation in alternative selection and mutation phases,
wherein associated with the mutation fermenter (fermenter 1) on the one hand and the selection fermenter (fermenter 2) on the other hand are computer-controlled regulating circuits by which the fermentation parameters in both fermenters are adjustable independently of each other.

2. A method according to claim 1 characterised in that the selection reactor operates toxinstatically.

3. A method according to claim 1 characterised in that the selection reactor operates turbidostatically.

4. A method according to claim 1, claim 2 or claim 3 characterised in that, in the transfer of biomass from one fermenter into the other, the base media of the biomass are exchanged.

5. A method according to claim 4 characterised in that exchange of the base media is effected in dialysis sections arranged between the fermenters.

6. A method according to one or more of the preceding claims characterised in that the base medium (base medium 1) of the mutation fermenter (fermenter 1) contains the harmful substance to be broken down.

7. A method according to claim 6 characterised in that the mutation fermenter (fermenter 1) operates toxinstatically.

8. A method according to claim 6 characterised in that the mutation fermenter (fermenter 1) operates turbidostatically.

## Revendications

1. Procédé pour la culture de microorganismes qui sont appropriés à la décomposition de matières nocives déterminées, où les microorganismes présents cultivés dans une solution nutritive provoquant la croissance sont soumis à des influences ayant un effet mutagène chimique et/ou physique, sont stabilisés dans des conditions provoquant la croissance et sont sélectionnés sous l'influence des matières nocives dans des conditions limitant la croissance et ces microorganismes sélectionnés sont ramenés au début du procédé, caractérisé en ce que la culture, le traitement mutagène et la stabilisation sont entrepris simultanément dans un fermenteur de mutation (fermenteur 1) qui contient un millier de base riche en aliments (milieu de base 1),
en ce que la sélection se produit dans un fermenteur de sélection (Fermenteur 2) qui contient un milieu de base pauvre en aliments, enrichi en matières nocives (milieu de base 2),
en ce que la biomasse est prélevée du fermenteur de mutation (Fermenteur 1) continuellement en petites quantités, laquelle est libérée du milieu de base riche en aliments (Milieu de base 1) du fermenteur de mutation (Fermenteur 1) et est conduite à un fermenteur de sélection (Fermenteur 2), et
en ce que, du fermenteur de sélection (Fermenteur 2), est prélevée continuellement, par petites quantités, la biomasse qui est libérée du milieu de base pauvre en aliments (Milieu de base 2) du fermenteur de sélection (Fermenteur 2) et est conduite dans le fermenteur de mutatior. (Fermenteur 1),
et en ce que tout le procédé se passe continuellement dans un circuit se répétant en phases alternées de sélection et mutation.
où au fermenteur de mutation (Fermenteur 1), d'une part et au fermenteur de sélection (Fermenteur 2), d'autre part, sont affectés des circuits de régulation commandés par ordinateur à travers lesquels les paramètres de fermentation dans les deux fermenteurs peuvent être réglés indépendamment les uns des autres.

2. Procédé selon la revendication 1, caractérisé en ce que le réacteur de sélection fonctionne en toxines statiques.

3. Procédé selon la revendication 1, caractérisé en ce que le réacteur de sélection fonctionne turbidostatiquement.

4. Procédé selon la revendication 1, 2 ou 3, caractérisé en ce que dans la transmission de la biomasse d'un fermenteur à l'autre, les milieux de base de la biomasse sont échangés.

5. Procédé selon la revendication 4, caractérisé en ce que l'échange des milieux de base est entrepris dans des tronçons de dialyse agencés entre les fermenteurs.

6. Procédé selon une ou plusieurs des revendications précédentes, caractérisé en ce que le milieu de base (Milieu de base 1) du fermenteur de mutation (Fermenteur 1) contient la matière nocive à décomposer.

7. Procédé selon la revendication 6, caractérisé en ce que le fermenteur de mutation (Fermenteur 1) fonctionne en toxines statiques.

8. Procédé selon la revendication 6, caractérisée en ce que le fermenteur de mutation (Fermenteur 1) fonctionne de façon turbidostatique.
